# EUROPEAN PATENT SPECIFICATION

(11) **EP 3 239 165 B1**
(45) Date of publication and mention of the grant of the patent: **09.10.2019**
(21) Application number: 15871972.4
(22) Date of filing: 24.12.2015
(51) Int. Cl.: C07K 1/30, C07K 7/56

(54) **COMPOSITION CONTAINING NITROGEN HETEROCYCLIC HEXAPEPTIDE PRECURSOR AND PREPARATION METHOD AND APPLICATION THEREOF**
ZUSAMMENSETZUNG MIT STICKSTOFFHALTIGEM HETEROCYCLISCHEM HEXAPEPTIDVORLÄUFER SOWIE HERSTELLUNGSVERFAHREN UND ANWENDUNG DAVON
COMPOSITION CONTENANT UN PRÉCURSEUR HEXAPEPTIDIQUE HÉTÉROCYCLIQUE D'AZOTE ET PROCÉDÉ DE PRÉPARATION ET APPLICATION ASSOCIÉS

(30) Priority: 24.12.2014 CN 201410840367
(43) Date of publication of application: 01.11.2017
(73) Proprietor: Shanghai Techwell Biopharmaceutical Co., Ltd, Shanghai 201108 (CN)
(72) Inventor: LIU, Shidong, Shanghai 201108 (CN); WANG, Xiusheng, Shanghai 201108 (CN); JI, Xiaoming, Shanghai 201108 (CN)
(74) Representative: Mewburn Ellis LLP
(86) International application number: PCT/CN2015/098740
(87) International publication number: WO 2016/101904

(56) References cited:
- WO-A1-94/21677
- WO-A1-2009/151341
- WO-A1-2011/102842
- CN-A- 102 295 686
- CN-A- 102 947 327
- PAN, YI ET AL.: 'STUDY ON PURIFICATION PROCESS OF PNEUMOCANDIN BO CHROMATOGRAPHY' BIOTECH WORLD 31 July 2012, XP009503935

## Description

### Technical field

The present invention relates to the field of pharmaceutics preparation and analysis of pharmaceutics, and relates to a composition of Pneumocandin B0 (which is a precursor compound of Caspofungin) and structural analogs thereof, and a preparation method thereof, as well as a method for preparing Caspofungin using the composition.

### Background

Pneumocandin B0, as shown in Formula I, is a secondary metabolite produced by microbial fermentation and has a cyclic hexapeptide structure. Pneumocandin B0 is generally used as a raw material for the synthesis of Caspofungin (Formula III), and preparation methods thereof are disclosed in a number of documents, such as U.S. Patent Nos. 5,594,377, 5,220,309 and 6,610,822.

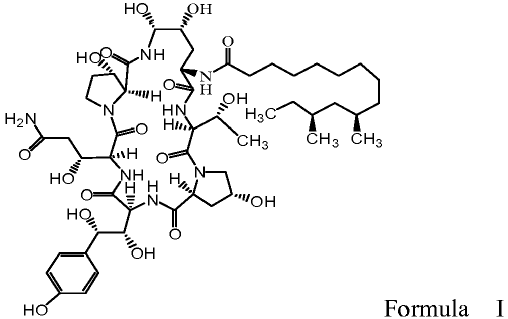

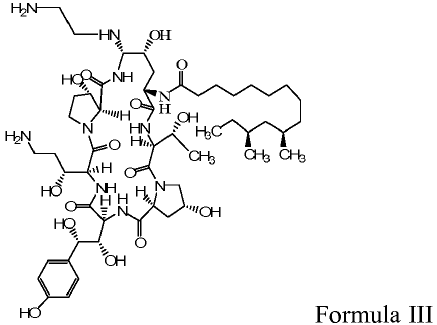

According to different substituents on proline in the structure of Pneumocandin, there are three main categories: A0, B0, and C0. According to different substituents on threonine, serine analogues, compounds of formula II with similar structures can be formed from B0.

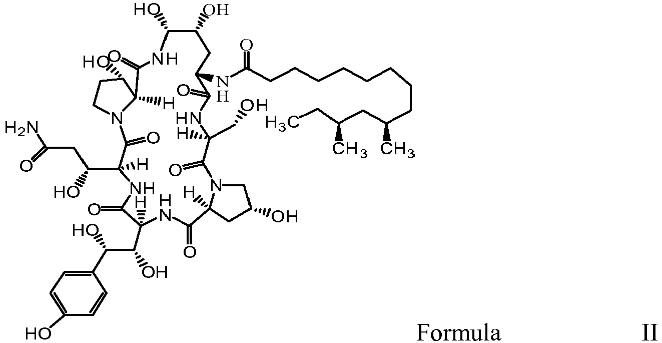

In the process of preparing the compound of formula I by fermentation, some impurities, such as Pneumocandin A0, C0 and serine analogs (with structure shown in formula II) are produced accordingly. Pneumocandin A0, C0 are separated and purified by means of methods such as extraction, precipitation, macroporous adsorption resin chromatography, preparative normal phase chromatography, and the method for separation and purification is described in WO2004042350A2, WO0220618A1, WO2005066323A1.

The compound of formula II and separation methods thereof are described in WO0008197A1 and U.S. Patent No. 5,378,8 04. WO-A1-9421677 discloses the compound caspofungin. WO-A1-2009151341 and WO-A1-2011102842 disclose the preparation of caspofungin from appropriate intermediates.

The compound of formula II can, to a certain extent, be removed by conventional separation and purification methods, for example using preparative normal phase chromatography, however effects are not satisfactory and the content of the compound of formula II in the final product is still very high (≥2.5%). And this method uses a large amount of organic solvents, causes serious environmental pollution, and is of high production cost, low production efficiency, and difficult to achieve large-scale industrial production.

It was described in Journal of Industrial Microbiology and Biotechnology, 26: p216-211, (2001) that serine analogs, impurity of formula II, contained in Pneumocandin B0 will be converted into serine analogue impurities of Caspofungin, resulting in a decrease in purity of the final product, caspofungin.

A compound of formula II contained in Pneumocandin B0 can be converted to a serine analog of caspofungin (e.g., formula IV) by, for example, the following reaction, during the synthesis of the compound of formula III:

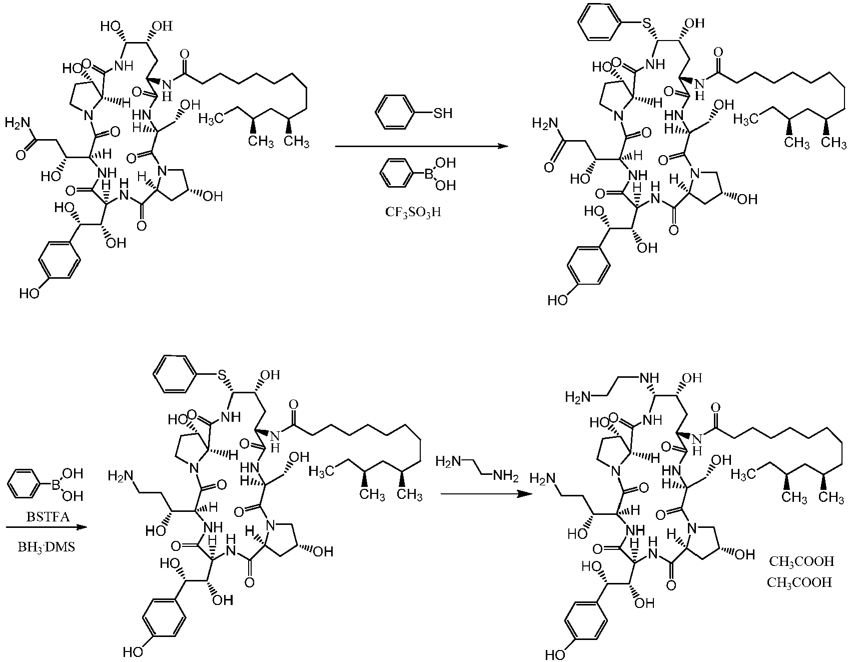

Relevant description also can be found in J. Org. Chem. 2007, 72, 2335-2343 233.

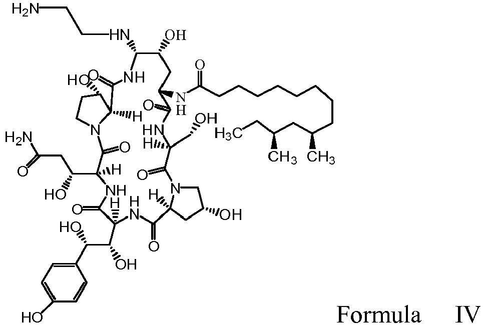

In CN102076707A, it was described that caspofungin acetate containing a compound of formula IV was separated and purified by using a medium to high pressure reverse phase preparative column, wherein RP C18 was used as a filler and acetonitrile / acetic acid solution was used as mobile phase, to prepare caspofungin acetate with low content of a compound of formula IV (serine analogue of Caspofungin). This process uses a large number of acetonitrile which is toxic and harmful, is of expensive production and can not be used for large-scale industrial production.

In CN102947327A, it was described that serine analogues contained in an intermediates was removed by using a normal phase silica gel preparative chromatographic column during intermediate stage for producing caspofungin. This purification process requires the use of a large amount of solvents, is of high production cost and basically of no value in industrial production.

Impurities are those that are not required in any active pharmaceutical ingredient (also known as API) and, in extreme cases, may even be harmful to patients who are receiving API-containing dosage forms.

The purity of API is a key point for its commercialization. FDA explicitly requires that impurities must be controlled below a specified limit during the process of producing API. In ICH Q7A guidelines, FDA specifies the quality of raw material that can be used, as well as acceptable process conditions such as temperature, pressure, time and stoichiometric ratios, including purification steps such as crystallization, distillation and liquid-liquid extraction. (See ICH Good Manufacturing Practice Guide for Active Pharmaceutial Ingredients, Q7A). A product from a chemical reaction is rarely a single compound with sufficient purity which meets the standard for a drug. In most cases, by-products from the reaction and the adjuvants used in the reaction will also be present in the product. During certain stages of processing API, the purity thereof must be analyzed, usually by high performance liquid chromatography or thin layer chromatography, to determine whether it is suitable for subsequent processing and ultimate use in pharmaceutical products. FDA requires that impurities in API should be minimized, thereby maximizing the safety when an API is clinically used. For example, FDA recommends that the amount of certain impurities be limited to less than 0.1 percent (see ICH Pharmaceutical Product Quality Management Practices for Active Pharmaceutical Ingredients, Q7A).

According to the above requirements on the limit of impurities in API, how to improve the purity of API as much as possible and reduce the content of impurities become a key point for developing a process. If a final drug product is too difficult to be purified, it is generally considered to improve its purity by increasing the purity of its intermediates. Therefore, in the preparation process of the compound of formula III, the higher the purity of the compound of formula I as an intermediate, the higher the purity of the compound of formula III obtained by chemical modification. If the chemically modified compound of formula III is of high purity, it is possible to greatly reduce the pressure on the purification step of the compound of formula III and obtain the final drug product of high purity using a simple purification process. Therefore, the content of the compound of formula II is required to be as low as possible.

Therefore, it is an urgent desire for the inventors to obtain Pneumocandin B0 with very low content of the compound of formula II by using a specific purification means during Pneumocandin B0 stage.

### Summary of the Invention

Initially, the inventors have attempted to remove the impurity, the compound of formula II, by conventional crystallization methods to obtain crystals of the compound of formula I. However, after extensive experiments, it has been found that in crystals of the compound of formula I obtained by conventional crystallization means, such as by adding an anti-solvent, the content of the compound of formula II is not reduced; in other words, there is no effect on removing the compound of formula II by conventional crystallization process.

After a large number of crystallization experiments, the inventors unexpectedly found that, in some experiments, after an organic solvent was added, the solution will be turbid, and solid particles obviously precipitated. However, the solid particles, unlike crystals, do not precipitate, but are suspended in a mixed liquid. The solid particles were isolated from the mixture by the inventors, and after further analysis, it was surprisingly found that the solid particles were compounds of formula I in non-crystalline form and the content of the impurity (compound of formula II) was extremely low. The above results were confirmed by repeated experiments, therefore, it can be concluded that the impurity (the compound of formula II) can be effectively removed by obtaining the above-mentioned suspension system. After further study on the above system, it was confirmed by the inventors that: the above suspension is actually a suspension of a crude dispersion system formed by the compound of formula I with water and solvents (see "Colloid and Interface Chemistry", Higher Education Press), that is, a special state formed by the compound of formula I, water and solvents with a particle diameter being greater than 0.1 µm. In this state, the compound of formula I is not dissolved in solvents to form a solution, and does not precipitate in a form of precipitates or crystals, but is suspended in solvents on a form of particles. However, the compound of formula II is dissolved in solvents in a large amount and the solid particles and solvents can be separated by filtration or centrifugation to obtain the compound of formula I of higher purity, while a large amount of the compound of formula II is kept in the solvent, so that the impurity, the compound of formula II, can be efficiently removed from the compound of formula I.

In order to determine the method for obtaining the above suspension of crude dispersion system, the inventors have conducted extensive experimental studies and found that: when the moisture content of the solvent system is controlled at 8% - 30% by volume, a suspension of crude dispersion system can be obtained. When the moisture content is higher than 30%, the moisture content is too high, and when a non-polar anti-solvent is added, the amount of the anti-solvent is usually too large or the whole system is separated, and the crude dispersion system can not be finally obtained.

In addition, according to the experimental data, the content of the impurity, the compound of formula II can be reduced by 40% to 60% for every cycle of the above operations, while the loss of the compound of formula I is only 2% or less. By repeating the above method, the content of the impurity, the compound of formula II in the compound of formula I can be reduced to 2.0%, preferably 0.49% or less; more preferably 0.2% or less; and most preferably 0.1% or less.

Further, when the compound of formula I with a very low content of the compound of formula II is used as a raw material for the preparation of caspofungin or caspofungin acetate, the content of caspofungin serine analog impurity (the compound of formula IV) in the obtained caspofungin (the compound of formula III) can be generally controlled at 0.1% or less, thereby greatly reducing the pressure on the purification phase of caspofungin.

### Process for preparing a composition comprising the compound of formula I and formula II and compositions A and B obtained by the process

A process for preparing a composition comprising a compound of formula I and a compound of formula II is provided in the present invention, comprising steps of:
(a) dissolving the crude compound of formula I in an aqueous solution of an organic solvent (i);
(b) precipitating solids from the solution by cooling and / or adding an organic solvent (ii) to obtain a suspension containing the compound of formula I;
(c) obtaining composition A containing the compound of formula I and the compound of formula II by centrifugation or filtration.

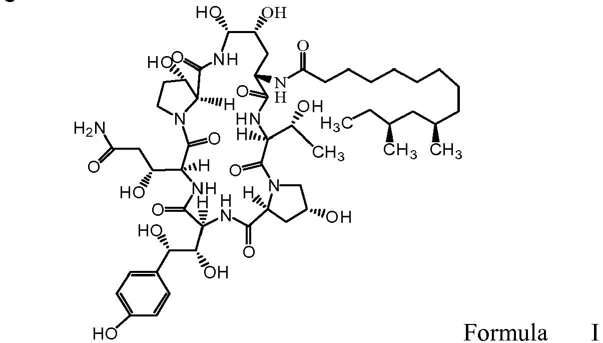

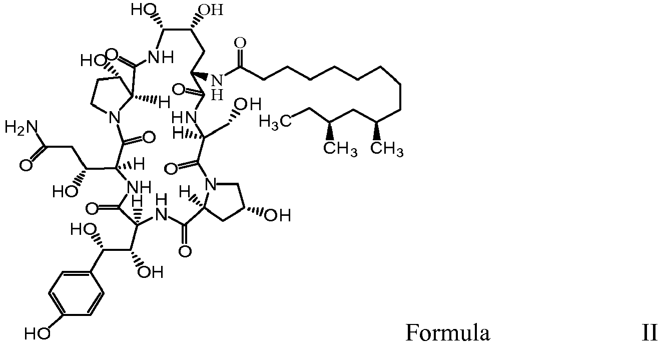

In another preferred embodiment, in step (a), the volume percentage of water in the aqueous solution of organic solvent (i) is from 8% to 30%, preferably from 10% to 25%, more preferably from 12% to 22%, and most preferably from 16% to 22%.

In step (a), the organic solvent (i) is selected from one or more of methanol, ethanol, n-propanol, isopropanol, isobutanol, n-butanol, and acetone.

In step (b), the organic solvent (ii) is selected from one or more of C₃₋₇ ester, hexane, n-heptane, n-pentane, dichloromethane; and preferably from one or more of ethyl acetate, isopropyl acetate, n-hexane.

In another preferred embodiment, the steps (a) - (c) may be repeated one or more times.

A suspension containing the compound of formula I obtained by the above-mentioned process is also provided in the present invention, and the diameter of the solid particles in the suspension is greater than or equal to 0.1 µm.

In a preferred embodiment, the diameter of the solid particles in the suspension is greater than or equal to 0.2 µm.

In another preferred embodiment, the diameter of the solid particles in the suspension is greater than or equal to 0.3 µm.

In a preferred embodiment, the diameter of solid particles in the suspension, i.e., "particle size distribution", is determined using Malvern particle size meter 2600C. A preferred method for determining the diameter of solid particles in a suspension is laser diffraction.

Composition A comprising a compound of formula I and a compound of formula II obtained by the above-mentioned process is also provided in the present invention, wherein the composition further comprises from 1% to 40% of an organic solvent (mass percent) and 1% to 15% water (mass percent).

In a preferred embodiment, the composition also comprises 5% to 35% of organic solvent (mass percent) and 2% to 12% of water (mass percent).

In another preferred embodiment, the composition further comprises 10-30% of organic solvent (mass percent) and 3% to 10% of water (mass percent).

In another preferred embodiment, the organic solvent is selected from a group consisting of methanol, ethanol, n-propanol, isopropanol, isobutanol, n-butanol, acetone, ethyl acetate, isopropyl acetate, n-hexane, dichloromethane.

In another preferred embodiment, in composition A, the dry content of the compound of formula I is more than 95%, and the dry content of the compound of formula II is from 0.0001% to 2.0%; preferably from 0.0001% to 0.49 %; more preferably from 0.0001% to 0.2%; and most preferably from 0.0001% to 0.1%.

In another preferred embodiment, in composition A, the dry content of each component is determined by HPLC assay.

In another preferred embodiment, the HPLC assay is:
Chromatographic Column: Symmtry C18 3.5um 2.1 × 150 mm;
Mobile phase: acetonitrile / water = 39/61;
Flow rate: 0.4 ml / min;
Column temperature: 30°C;
Sample diluent: acetonitrile / water = 39/61;
Injection temperature: 5°C;
Detection wavelength: 205nm.

In another preferred embodiment, composition A containing a compound of formula I and a compound of formula II is further dried to obtain a dry composition B.

In another preferred embodiment, the moisture content of composition B is less than or equal to 5%.

### Composition B comprising a compound of formula I and a compound of formula II

Composition B comprising a compound of formula I and a compound of formula II is provided in the present invention.

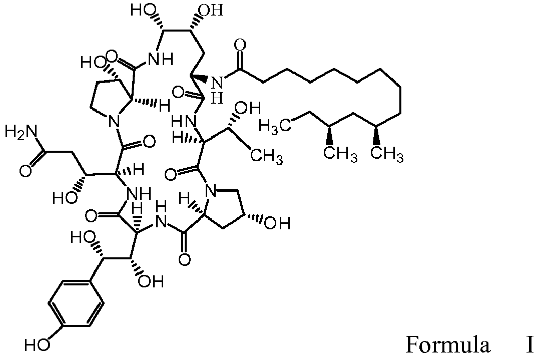

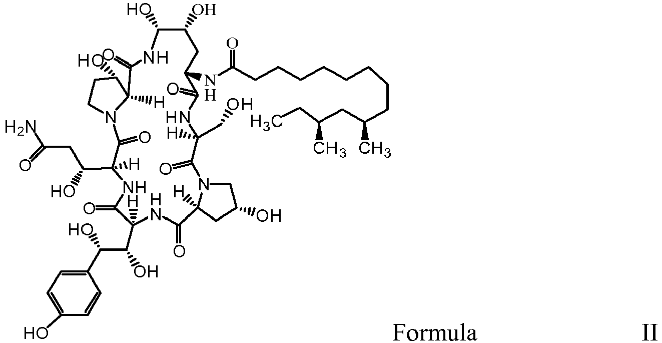

In another preferred embodiment, the dry content of the compound of formula I is more than 95%.

In another preferred embodiment, the dry content of the compound of formula II is from 0.0001% to 2.0%.

In another preferred embodiment, the dry content of the compound of formula II is from 0.0001% to 0.49 %.

In another preferred embodiment, the dry content of the compound of formula II is from 0.0001% to 0.2%.

In another preferred embodiment, the dry content of the compound of formula II is from 0.0001% to 0.1%.

In another preferred embodiment, the dry content of each component in the composition is determined by HPLC assay.

In another preferred embodiment, the HPLC assay is:
Chromatographic Column: Symmtry C18 3.5um 2.1 × 150 mm;
Mobile phase: acetonitrile / water = 39/61;
Flow rate: 0.4 ml / min;
Column temperature: 30°C;
Sample diluent: acetonitrile / water = 39/61;
Injection temperature: 5°C;
Detection wavelength: 205nm.

### Use of Composition B comprising a compound of Formula I and a compound of Formula II

Use of composition B comprising a compound of formula I and a compound of formula II is also provided in the present invention for preparing a compound of formula X.

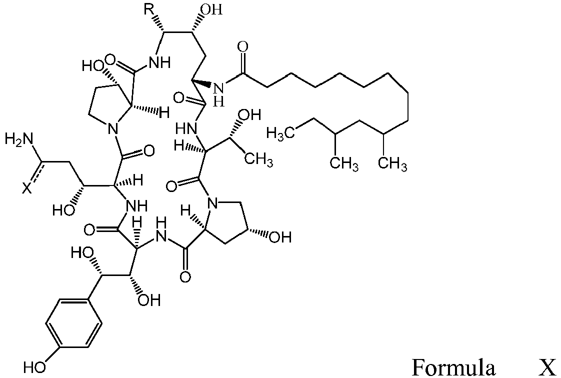

| Compound X | R | X (a=O, b=2H) |
|---|---|---|
| X1 | 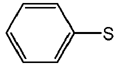 | a |
| | | b |
| X2 | 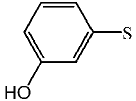 | a |
| | | b |
| X3 | 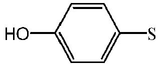 | a |
| | | b |
| X4 | 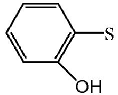 | a |
| | | b |
| X5 | 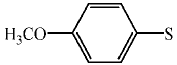 | a |
| | | b |
| X6 | 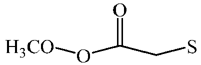 | a |
| | | b |
| X7 | 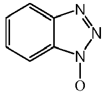 | a |
| | | b |
| X8 | 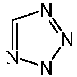 | a |
| | | b |
| X9 | 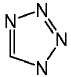 | a |
| | | b |
| X10 | 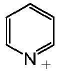 | a |
| | | b |
| X11 | 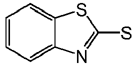 | a |
| | | b |
| X12 | 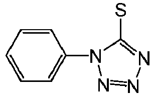 | a |
| | | b |

### Composition comprising a compound of formula X and a compound of formula XI

A composition comprising a compound of formula X and a compound of formula XI is also provided in the present invention.

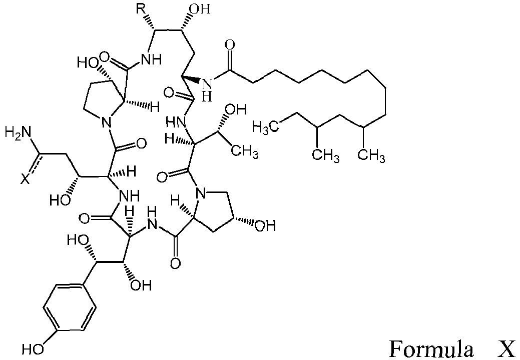

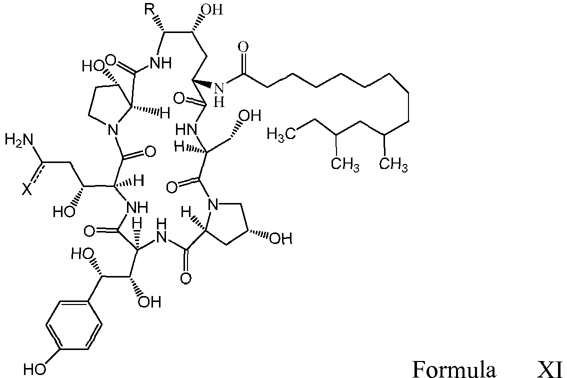

| Compound X | Compound XI | R | X (a=O, b=2H) |
|---|---|---|---|
| X1 | XI1 | 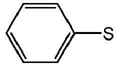 | a |
| | | | b |
| X2 | XI2 | 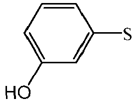 | a |
| | | | b |
| X3 | XI3 | 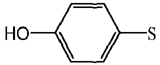 | a |
| | | | b |
| X4 | XI4 | 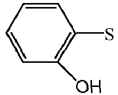 | a |
| | | | b |
| X5 | XI5 | 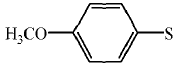 | a |
| | | | b |
| X6 | XI6 | 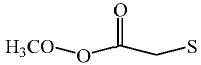 | a |
| | | | b |
| X7 | XI7 | 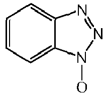 | a |
| | | | b |
| X8 | XI8 | 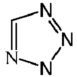 | a |
| | | | b |
| X9 | XI9 | 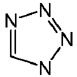 | a |
| | | | b |
| X10 | XI10 | 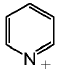 | a |
| | | | b |
| X11 | XI11 | 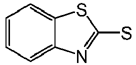 | a |
| | | | b |
| X12 | XI12 | 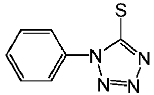 | a |
| | | | b |

In another preferred embodiment, the content of the compound of formula X in the composition is more than 95%.

In another preferred embodiment, the HPLC content of the compound of formula XI in the composition is from 0.0001% to 2.0%.

In another preferred embodiment, the HPLC content of the compound of formula XI in the composition is from 0.0001% to 0.49%.

In another preferred embodiment, the HPLC content of the compound of formula XI in the composition is from 0.0001% to 0.2%.

In another preferred embodiment, the HPLC content of the compound of formula XI in the composition is from 0.0001% to 0.1%.

### Use of a composition comprising a compound of formula X and a compound of formula XI

Use of the composition comprising a compound of formula X and a compound of formula XI is also provided in the present invention for preparing a compound of formula III.

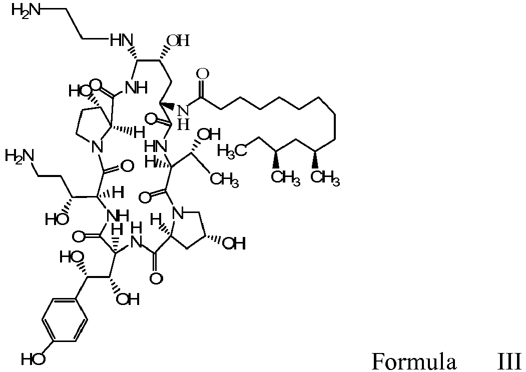

### Related terms

As used herein, "formula I compound", "compound of formula I", "Pneumocandin B0" may be interchangeably used and refer to a compound with a chemical structure as shown in formula I.

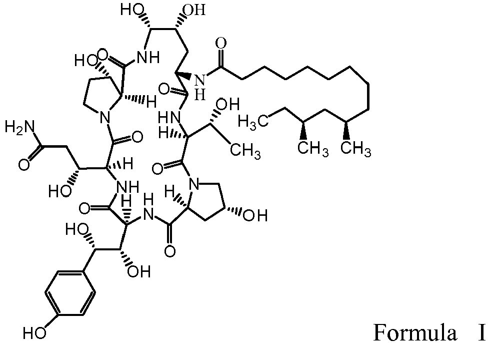

As used herein, "crude compound of formula I", "crude Pneumocandin B0" refer to a raw material containing a compound of formula I, which can be obtained by conventional methods in the art, for example, but not limited to, methods for preparing the compound of formula I disclosed in U.S. Patent Nos. 5,194,377, 5,220,309, 6,620,822 and WO2000/008197; and may also be commercially obtained from, for example, but not limited to, Merck. According to different fermentation processes provided in the above-mentioned patents, we have studied the contents of impurity for different fermentation process, and it was confirmed that the content of the compound of formula II in the compound of formula I obtained from different fermentation processes was from 0.5% to 8%.

As used herein, "formula II compound", "compound of formula II", "serine analog impurity" may be interchangeably used and refer to a compound having a structure shown in formula II, which is an impurity structurally similar to the compound of formula I.

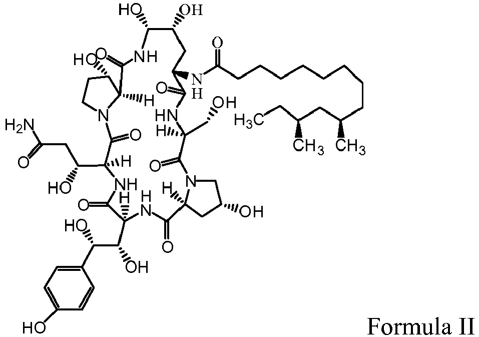

As used herein, "formula III compound", "compound of formula III" or "caspofungin" may be interchangeably used and refer to a compound having a chemical structure shown in formula III, and an acetate thereof can be used as an antifungal agent for treating invasive aspergillosis, esophageal candidiasis, intraperitoneal abscess caused by *Candida,* pleurisy, abdominal infection, and fever caused by unidentified pathogens in a patient with neutropenia, and so on.

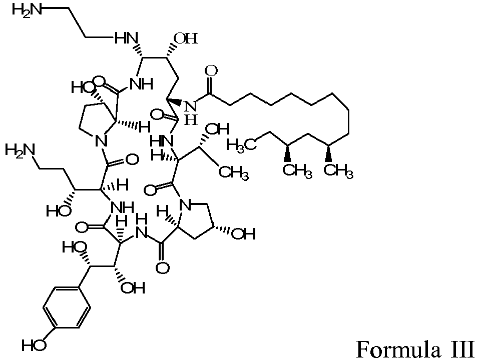

As used herein, "formula X compound", "compound of formula X" may be interchangeably used and refer to a compound having a chemical structure as shown in formula X; and "formula XI compound", "compound of formula XI" may be interchangeably used and refer to a compound having a chemical structure as shown in Formula XI; wherein X is O or 2H.

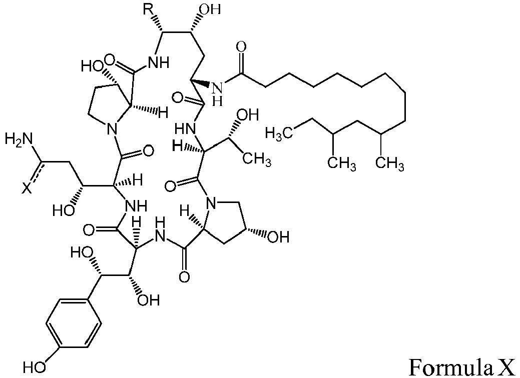

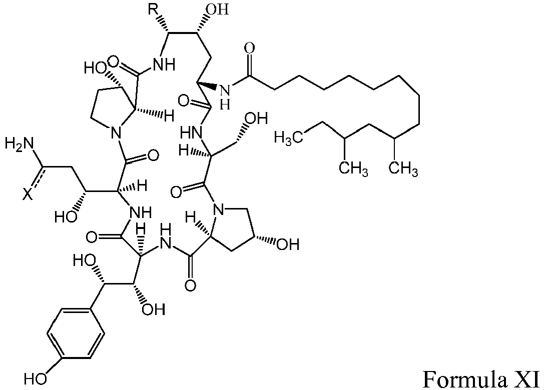

| Compound X | Compound XI | R | X (a=O, b=2H) |
|---|---|---|---|
| X1 | XI1 | 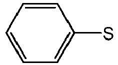 | a |
| | | | b |
| X2 | XI2 | 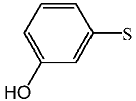 | a |
| | | | b |
| X3 | XI3 | 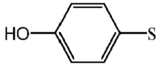 | a |
| | | | b |
| X4 | XI4 | 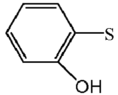 | a |
| | | | b |
| X5 | XI5 | 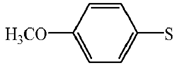 | a |
| | | | b |
| X6 | XI6 | 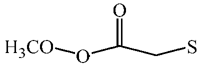 | a |
| | | | b |
| X7 | XI7 | 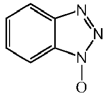 | a |
| | | | b |
| X8 | XI8 | 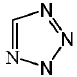 | a |
| | | | b |
| X9 | XI9 | 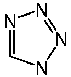 | a |
| | | | b |
| X10 | XI10 | 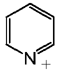 | a |
| | | | b |
| X11 | XI11 | 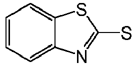 | a |
| | | | b |
| X12 | XI12 | 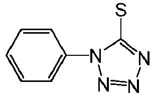 | a |
| | | | b |

The compound of formula X is prepared from the compound of formula I as a raw material and can be obtained by conventional methods in the art, for example, but not limited to, preparation methods disclosed in J. Org. Chem. 2007, 72, 2335-2343 and the like. The compound of formula III can be prepared by conventional methods from the compound of formula X, for example, but not limited to, preparation methods disclosed in J. Org. Chem. 2007, 72, 2335-2343 and the like.

### Description of Drawings

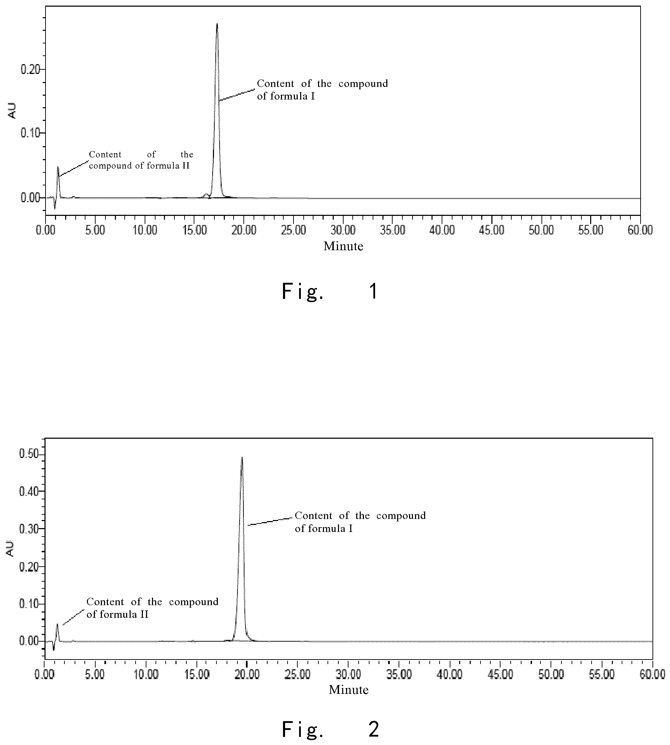

Figure 1 shows a HPLC chromatogram of the crude compound of formula I obtained in Example 1.
Figure 2 shows a HPLC chromatogram of composition A10 obtained in Example 6.

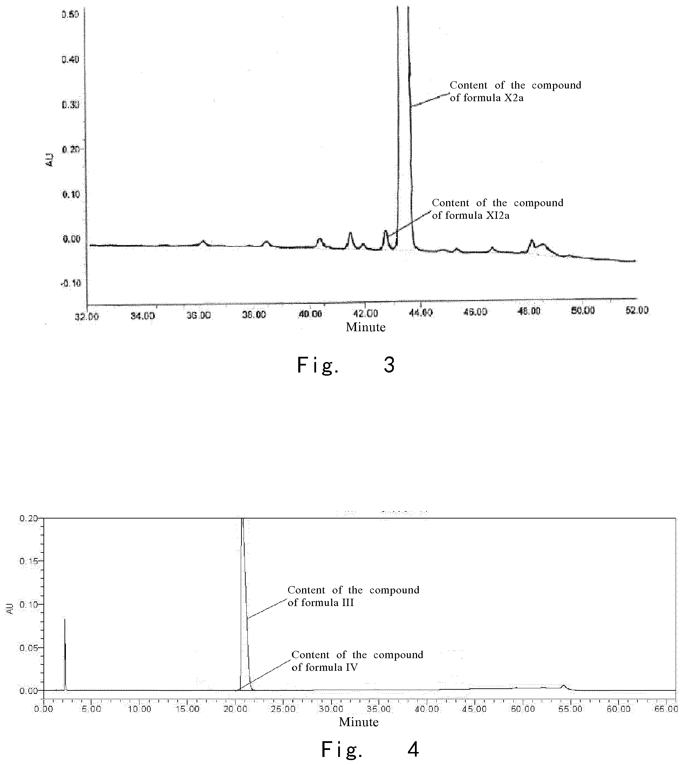

Figure 3 shows a HPLC chromatogram of the compound of formula X2a obtained in Example 10.
Figure 4 shows a HPLC chromatogram of the compound of formula III obtained from Example 18 (caspofungin).

### Modes for carrying out the invention

The invention will now be further described with reference to specific embodiments. It is to be understood that these examples are merely illustrative of the invention and are not intended to limit the scope of the invention. The experimental methods not specified in the following examples are generally prescribed in accordance with conventional conditions or in accordance with the conditions recommended by the manufacturer. Unless otherwise stated, all percentages, ratios, proportions, or parts are by weight.

Unless otherwise defined, all professional and scientific terms used herein are of the same meaning as those skilled in the art. In addition, any method and material similar to or equivalent to the described contents may be applied to the method of the present invention. The preferred embodiments and materials described herein are for exemplary purposes only.

The contents of Pneumocandin B0 and serine analogs are analyzed using the following HPLC assay:
Chromatographic Column: Waters Symmtry C18 3.5um 2.1 × 150mm;
Mobile phase: acetonitrile / water = 39/61;
Flow rate: 0.4 ml / min;
Column temperature: 30°C;
Sample diluent: acetonitrile / water = 39/61;
Injection temperature: 5°C;
Detection wavelength: 205nm.

The contents of caspofungin intermediate and serine analogs thereof are analyzed using the following HPLC assay:
Chromatographic Column: YMC-Pack ODS-A 250 × 4.6mm, 5 um;
Mobile phase A: 0.1% perchloric acid and 0.075% aqueous solution of sodium chloride;
Mobile phase B: acetonitrile;
Flow rate: about 1.5ml / min;
Column temperature: 30°C;
Detection wavelength: 220nm;

**Gradient procedure:**

| Time (min) | %A | %B |
|---|---|---|
| 0 | 65.5 | 34.5 |
| 14.5 | 65.5 | 34.5 |
| 35 | 50 | 50 |
| 45 | 35 | 65 |
| 50 | 20 | 80 |
| 52 | 20 | 80 |
| 53 | 65.5 | 34.5 |
| 66 | 65.5 | 34.5 |

### Example 1 (Preparation of crude compound of formula I)

According to WO2000/008197, a crude product containing the compound of formula I was obtained by the fermentation process of Glarea Lozoyensis (Zalerion arboricla).

According to WO2005026323, the above obtained crude product containing the compound of formula I was purified by chromatography and re-fined fractions were collected, so as to obtain 267 g of the compound of formula I. The content of the compound of formula II was determined as 3.1%.

### Example 2 (Preparation of a crude product of the compound of formula I)

According to "Pneumocandin B0 Production by Fermentation of the Fungus Glarea lozoyensis: Physiological and Engineering Factors Affecting Titer and Structural Analogue Formation", the crude product of the compound of formula I was obtained by adding serine during fermentation.

According to WO2005026323, the above obtained crude product containing the compound of formula I was purified by chromatography and re-fined fractions were collected, so as to obtain 36 g of the compound of formula I. The content of the compound of formula II was determined as 8.0%.

### Example 3 (Preparation of a crude product of compound of formula I)

According to WO2005026323, threonine was added during fermentation to give a crude product of the compound of formula I. The above obtained crude product containing the compound of formula I was purified by chromatography and re-fined fractions were collected, so as to obtain 22 g of the compound of formula I. The content of the compound of formula II was determined as 0.5%.

### Example 4

36 g of the crude product of the compound of formula I obtained in Example 1, in which the content of the compound of formula II was 3.1%, was dissolved in an isobutanol solution having a moisture content of 18% and a volume of 720 ml. 1440 ml of ethyl acetate was slowly added and stirred to obtain a suspension containing the compound of formula I. The diameter of solid particles of the compound of formula I in the suspension were determined as 0.3um or higher using Malvern particle size meter 2600C. Composition A was obtained through solid-liquid separation by filtration, and the loss of the compound of formula I was 1.5%. The content of the compound of formula I in composition A1 was determined as 98.6% and the content of compound of formula II was determined as 0.95% by HPLC.

The above-obtained composition A1 (content of the compound of formula II was 0.95%) was divided into two portions. One portion was dried *in vacuo* to give 17.6 g of composition B1, the moisture content was determined as 4%, and the content of the compound of formula II was determined as 0.95%.

Another portion of composition A1 was dissolved in an isobutanol solution having a moisture content of 12% in a volume of 240 ml. 460 ml of isopropyl acetate was slowly added and stirred to obtain a suspension containing the compound of formula I. The diameter of solid particles of the compound of formula I in the suspension were determined as 0.5um or higher using Malvern particle size meter 2600C. Composition A2 was obtained through solid-liquid separation by filtration, and the loss of the compound of formula I in the liquid was 1.6%. The content of compound of formula II was determined as 0.38% by HPLC.

Composition A2 (in which the content of the compound of formula II was 0.38%) above-obtained by filtration was dissolved in an iso-butanol solution having a moisture content of 20% in a volume of 246 ml. 460 ml of isopropyl acetate was slowly added and stirred to obtain a suspension containing the compound of formula I. The diameter of solid particles of the compound of formula I in the suspension were determined as 0.2um or higher using Malvern particle size meter 2600C. Composition A3 was obtained through solid-liquid separation by filtration, and the loss of the compound of formula I in the liquid was 1.2%. The content of compound of formula II was determined as 0.16% by HPLC.

Composition A3 (in which the content of the compound of formula II was 0.16%) above-obtained by filtration was dissolved in an iso-butanol solution having a moisture content of 15% in a volume of 206 ml. 410 ml of isopropyl acetate was slowly added and stirred to obtain a suspension containing the compound of formula I. The diameter of solid particles of the compound of formula I in the suspension were determined as 0.4um or higher using Malvern particle size meter 2600C. Composition A4 was obtained through solid-liquid separation by filtration, and the loss of the compound of formula I in the liquid was 1.0%. The content of compound of formula II was determined as 0.09% by HPLC.

The above composition A4 was dried *in vacuo* to give 17 g of composition B2, the moisture content was determined as 2.4%, and the content of the compound of formula II was determined as 0.09%.

### Example 5

6 g of the crude product of the compound of formula I obtained in Example 2, in which the content of the compound of formula II was 8%, was dissolved in an isobutanol solution having a moisture content of 8% and a volume of 150 ml. 250 ml of isopropyl acetate was slowly added and stirred to obtain a suspension containing the compound of formula I. The diameter of solid particles of the compound of formula I in the suspension were determined as 0.2um or higher using Malvern particle size meter 2600C. Composition A5 was obtained through solid-liquid separation by filtration, and the loss of the compound of formula I in the liquid was 1.9%. The content of the compound of formula I in composition A5 was determined as 96.4% and the content of compound of formula II was determined as 3.1% by HPLC.

Composition A5 (in which the content of the compound of formula II was 3.1%) above-obtained by filtration was dissolved in an iso-butanol solution having a moisture content of 12% in a volume of 120 ml. 230 ml of isopropyl acetate was slowly added and stirred to obtain a suspension containing the compound of formula I. The diameter of solid particles of the compound of formula I in the suspension were determined as 0.3um or higher using Malvern particle size meter 2600C. Composition A6 was obtained through solid-liquid separation by filtration, and the loss of the compound of formula I in the liquid was 1.6%. The content of compound of formula II was determined as 1.4% by HPLC.

Composition A6 (in which the content of the compound of formula II was 1.4%) above-obtained by filtration was dissolved in an iso-butanol solution having a moisture content of 20% in a volume of 123 ml. 230 ml of isopropyl acetate was slowly added and stirred to obtain a suspension containing the compound of formula I. The diameter of solid particles of the compound of formula I in the suspension were determined as 0.4um or higher using Malvern particle size meter 2600C. Composition A7 was obtained through solid-liquid separation by filtration, and the loss of the compound of formula I in the liquid was 1.2%. The content of compound of formula II was determined as 0.6% by HPLC.

Composition A7 (in which the content of the compound of formula II was 0.6%) above-obtained by filtration was dissolved in an iso-butanol solution having a moisture content of 15% in a volume of 103 ml. 205 ml of isopropyl acetate was slowly added and stirred to obtain a suspension containing the compound of formula I. The diameter of solid particles of the compound of formula I in the suspension were determined as 0.6um or higher using Malvern particle size meter 2600C. Composition A8 was obtained through solid-liquid separation by filtration, and the loss of the compound of formula I in the liquid was 1.0%. The content of compound of formula II was determined as 0.2% by HPLC.

Composition A8 (in which the content of the compound of formula II was 0.2%) above-obtained by filtration was dissolved in an iso-butanol solution having a moisture content of 15% in a volume of 80 ml. 190 ml of isopropyl acetate was slowly added and stirred to obtain a suspension containing the compound of formula I. The diameter of solid particles of the compound of formula I in the suspension were determined as 0.1um or higher using Malvern particle size meter 2600C. Composition A9 was obtained through solid-liquid separation by filtration, and the loss of the compound of formula I in the liquid was 1.0%. The content of compound of formula II was determined as 0.08% by HPLC.

The above composition A9 was dried *in vacuo* to give 5.3 g of composition B3, the moisture content was determined as 4.9%, and the content of the compound of formula II was determined as 0.08%.

### Example 6

9 g of the crude product of the compound of formula I obtained in Example 3, in which the content of the compound of formula II was 0.5%, was dissolved in an methanol-isobutanol solution (methanol: isobutanol = 2: 8 v/v) having a moisture content of 28% and a volume of 120 ml. 380 ml of ethyl acetate was slowly added and stirred to obtain a suspension containing the compound of formula I. The diameter of solid particles of the compound of formula I in the suspension were determined as 0.4um or higher using Malvern particle size meter 2600C. Composition A10 was obtained through solid-liquid separation by filtration, and the loss of the compound of formula I in the liquid was 1.9%. The content of the compound of formula I in composition A10 was determined as 98.8% and the content of compound of formula II was determined as 0.2% by HPLC.

Composition A10 (in which the content of the compound of formula II was 0.2%) above-obtained by filtration was dissolved in an ethanol solution having a moisture content of 12% in a volume of 120 ml. 230 ml of dichloromethane was slowly added and stirred to obtain a suspension containing the compound of formula I. The diameter of solid particles of the compound of formula I in the suspension were determined as 0.2um or higher using Malvern particle size meter 2600C. Composition A11 was obtained through solid-liquid separation by filtration, and the loss of the compound of formula I in the liquid was 1.6%. The content of compound of formula II was determined as 0.12% by HPLC.

Composition A11 (in which the content of the compound of formula II was 0.12%) above-obtained by filtration was dissolved in an isopropanol solution having a moisture content of 30% in a volume of 95 ml. 230 ml of n-hexane was slowly added and stirred to obtain a suspension containing the compound of formula I. The diameter of solid particles of the compound of formula I in the suspension were determined as 0.1um or higher using Malvern particle size meter 2600C. Composition A12 was obtained through solid-liquid separation by filtration, and the loss of the compound of formula I in the liquid was 0.8%. The content of compound of formula II was determined as 0.06% by HPLC.

Composition A12 (in which the content of the compound of formula II was 0.06%) above-obtained by filtration was dissolved in an n-butanol solution having a moisture content of 15% in a volume of 103 ml. 205 ml of ethyl acetate was slowly added and stirred to obtain a suspension containing the compound of formula I. The diameter of solid particles of the compound of formula I in the suspension were determined as 0.2um or higher using Malvern particle size meter 2600C. Composition A13 was obtained through solid-liquid separation by filtration, and the loss of the compound of formula I in the liquid was 1.0%. The content of compound of formula II was determined as 0.01% by HPLC.

The above composition A13 was dried *in vacuo* to give 8.6 g of composition B4, the moisture content was determined as 1.5%, and the content of the compound of formula II was determined as 0.01%.

### Comparative Example 1 (when the moisture content is controlled within 8%, the compound of formula I is in a crystalline state without impurity-removing effects)

2.6 g of the crude product of the compound of formula I obtained in Example 1, in which the content of the compound of formula II was 3.1%, was dissolved in a methanol solution having a moisture content of 7% and a volume of 22 ml. 100 ml of isopropyl acetate was added slowly, and solids precipitated. The solids were filtered and analyzed by HPLC to determine the content of the compound of formula II as 3.1% without any removing effect. X-ray powder diffraction (XRPD) analysis showed that the above precipitated solids were in crystalline form.

### Comparative Example 2 (when the moisture content is controlled within 8%, the compound of formula I is in a crystalline state without impurity-removing effects)

2.6 g of the crude product of the compound of formula I obtained in Example 1, in which the content of the compound of formula II was 3.1%, was dissolved in a n-propanol solution having a moisture content of 3% and a volume of 100 ml. 200 ml of ethyl acetate was added slowly, and solids precipitated. The solids were filtered and analyzed by HPLC to determine the content of the compound of formula II as 3.1% without any removing effect. X-ray powder diffraction (XRPD) analysis showed that the above precipitated solids were in crystalline form.

### Example 7 (preparing the compound of formula X1a from Composition B, wherein R is phenylthio)

Under an atmosphere of nitrogen, acetonitrile (300 ml), composition B2 (10.0 g, wherein the content of the compound of formula II was 0.09%), phenylboronic acid (2.0 g) and thiophenol (3.6 g) were homogeneously stirred and cooled to -20∼-15°C. Trifluoromethanesulfonic acid (2.5 ml) was added dropwise, and upon addition, the reaction was performed at -20∼-15°C for about 2.5 h. TLC showed that the reaction was completed. The reaction was quenched, and an aqueous NaOAc solution (2.3g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 125 ml of acetonitrile / water = 9: 1 (v / v) for three times and dried *in vacuo* for 5 h to obtain 9.0 g of sample containing the compound of formula X1a, and the content of the compound of formula XI1a was determined as 0.088% by HPLC.

### Example 8 (preparing the compound of formula X5a from Composition B, wherein R is methoxyphenylthio)

Under an atmosphere of nitrogen, acetonitrile (30 ml), composition B1 (1.0 g, wherein the content of the compound of formula II was 0.95%), phenylboronic acid (0.20 g) and methoxythiophenol (0.38 g) were homogeneously stirred and cooled to -20∼-15°C. Trifluoromethanesulfonic acid (0.25 ml) was added dropwise, and upon addition, the reaction was performed at -20∼-15°C for about 2.5 h. TLC showed that the reaction was completed. The reaction was quenched, and an aqueous NaOAc solution (0.23g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 0.94 g of sample containing the compound of formula X5a, and the content of the compound of formula XI5a was determined as 0.93% by HPLC.

### Example 9 (preparing the compound of formula X2a from Composition B, wherein R is m-hydroxyphenylthio)

Under an atmosphere of nitrogen, acetonitrile (30 ml), composition B4 (1.0 g, wherein the content of the compound of formula II was 0.01%), phenylboronic acid (0.2 g) and hydroxythiophenol (0.36 g) were homogeneously stirred and cooled to -20∼-15°C. Trifluoromethanesulfonic acid (0.25 ml) was added dropwise, and upon addition, the reaction was performed at -20∼-15°C for about 2.5 h. TLC showed that the reaction was completed. The reaction was quenched, and an aqueous NaOAc solution (0.23g NaOAc dissolved in 0.5ml of water) was slowly added. Upon addition, the temperature was raised to 20°C and the reaction system was stirred for 2h. Large amount of solids precipitated, cooled to below 0°C, and filtered. The filter cake was washed with 1.25 ml of acetonitrile / water = 9: 1 (v / v) for three times and dried *in vacuo* for 5 h to obtain 0.93 g of sample containing the compound of formula X2a, and the content of the compound of formula XI2a was determined as 0.009% by HPLC.

### Example 10 (preparing the compound of formula X2a from Composition B, wherein R is m-hydroxyphenylthio)

Under an atmosphere of nitrogen, acetonitrile (300 ml), composition B1 (10 g, wherein the content of the compound of formula II was 0.95%), phenylboronic acid (2.0 g) and *m*-hydroxythiophenol (3.6 g) were homogeneously stirred and cooled to -20∼-15°C. Trifluoromethanesulfonic acid (2.5 ml) was added dropwise, and upon addition, the reaction was performed at -20∼-15°C for about 2.5 h. TLC showed that the reaction was completed. The reaction was quenched, and an aqueous NaOAc solution (2.3g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 9.3 g of sample containing the compound of formula X2a, and the content of the compound of formula XI2a was determined as 0.87% by HPLC.

### Example 11 (preparing the compound of formula X2a from Composition B, wherein R is m-hydroxyphenylthio)

Under an atmosphere of nitrogen, acetonitrile (300 ml), composition B2 (10 g, wherein the content of the compound of formula II was 0.09%), phenylboronic acid (2.0 g) and m-hydroxythiophenol (3.6 g) were homogeneously stirred and cooled to -20∼-15°C. Trifluoromethanesulfonic acid (2.5 ml) was added dropwise, and upon addition, the reaction was performed at -20∼-15°C for about 2.5 h. TLC showed that the reaction was completed. The reaction was quenched, and an aqueous NaOAc solution (2.3g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 9.2 g of sample containing the compound of formula X2a, and the content of the compound of formula XI2a was determined as 0.09% by HPLC.

**Example 12 (preparing the compound of formula X9a from Composition B, wherein R is** 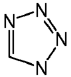 )

Under an atmosphere of nitrogen, acetonitrile (30 ml), composition B3 (1.0 g, wherein the content of the compound of formula II was 0.08%), phenylboronic acid (0.20 g) and Tetrazole (0.27 g) were homogeneously stirred and cooled to -20∼-15°C. Trifluoromethanesulfonic acid (0.25 ml) was added dropwise, and upon addition, the reaction was performed at -20∼-15°C for about 2.5 h. TLC showed that the reaction was completed. The reaction was quenched, and an aqueous NaOAc solution (0.23g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 0.91 g of sample containing the compound of formula X9, and the content of the compound of formula XI9a was determined as 0.079% by HPLC.

**Example 13 (preparing the compound of formula X10 from Composition B, wherein R is** 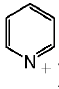 )

Under an atmosphere of nitrogen, acetonitrile (30 ml), composition B3 (1.0 g, wherein the content of the compound of formula II was 0.08%), phenylboronic acid (0.20 g) and pyridine (0.32 g) were homogeneously stirred and cooled to -20∼-15°C. Trifluoromethanesulfonic acid (0.25 ml) was added dropwise, and upon addition, the reaction was performed at -20∼-15°C for about 2.5 h.

TLC showed that the reaction was completed. The reaction was quenched, and an aqueous NaOAc solution (0.23g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 0.90 g of sample containing the compound of formula X10, and the content of the compound of formula XI10a was determined as 0.079% by HPLC.

**Example 14 (preparing the compound of formula X11a from Composition B, wherein R is** 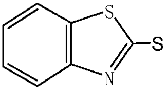 )

Under an atmosphere of nitrogen, acetonitrile (30 ml), composition B4 (1.0 g, wherein the content of the compound of formula II was 0.01%), phenylboronic acid (0.20 g) and mercaptobenzothiazole (0.45 g) were homogeneously stirred and cooled to -20∼-15°C. Trifluoromethanesulfonic acid (0.25 ml) was added dropwise, and upon addition, the reaction was performed at -20∼-15°C for about 2.5 h. TLC showed that the reaction was completed. The reaction was quenched, and an aqueous NaOAc solution (0.23g NaOAc dissolved in 5ml of water) was slowly added. Upon addition, the temperature was raised to 20°C and the reaction system was stirred for 2h. Large amount of solids precipitated, cooled to below 0°C, and filtered. The filter cake was washed with 12.5 ml of acetonitrile / water = 9: 1 (v / v) for three times and dried *in vacuo* for 5 h to obtain 0.98 g of sample containing the compound of formula X11, and the content of the compound of formula XI11a was determined as 0.009% by HPLC.

### Example 15 (reduction reaction of the compound of formula X1a)

Under an atmosphere of nitrogen, the compound of formula X1a (2.0 g) obtained in Example 7, phenylboronic acid (0.28 g) and anhydrous tetrahydrofuran (80 ml) were heated under reflux for 30 min, and cooled to room temperature. BSTFA (2.1 ml) was added, stirred for 1h at room temperature, and cooled to -10∼-5°C. Borane-dimethyl sulfide complex (0.8ml, 0.94%) was added dropwise, and upon addition, warmed to 10∼15°C for reacting for 3.5h. The reaction was monitored by HPLC and the conversion rate was 82%. Afterwards, hydrochloric acid (2 N, 4.8 ml) was added dropwise and water (160 ml) was added. The solvent was removed under reduced pressure and then stirred at room temperature for 24 h. The residue was loaded onto a preparative column, and eluted with 22% acetonitrile / water (0.15% acetic acid). Collections rich in products were pooled, diluted twice with water, loaded on a preparative column, and eluted with 90% acetonitrile / water (0.15% acetic acid). Fractions were collected and lyophilized to give 1.6 g of sample containing the compound of formula X1b. The content of the compound of formula XI1b was determined as 0.082% by HPLC.

### Example 16 (Preparing the compound of formula III from the compound of formula X1b)

Under an atmosphere of nitrogen, the compound of formula X1b (1.0 g) obtained in Example 15 was dissolved in methanol (4.2 ml), and cooled to -20 to -15°C. Ethylenediamine (4.2 ml) was added dropwise. Upon addition, the reaction was warmed to room temperature for 48 h, and the reaction conversion rate was determined as 99% by HPLC monitoring. The reaction system was added dropwise into a solution of glacial acetic acid solution (8.3 ml) in water (18.5 ml), then diluted twice with water, loaded onto a preparative column and eluted with 22% acetonitrile / water (0.15% acetic acid). Collections rich in products were pooled, diluted twice with water, loaded on a preparative column, and eluted with 90% acetonitrile / water (0.15% acetic acid). Fractions were collected and concentrated to dryness under reduced pressure. The above concentrate was dissolved in a solution (ethanol / water / acetic acid = 210.0/19.7/1.0, v/v/v, 10 ml), and ethyl acetate (14 ml) was added dropwise at 5 to 20°C for crystallization. White crystalline solids (caspofungin acetate, 0.71 g) were obtained by filtration, the purity of which was determined as 99.82% by HPLC, and the content of the compound of formula IV was 0.072%.

### Example 17 (reduction reaction of the compound of formula X2a)

Under an atmosphere of nitrogen, the compound of formula X2a (2.0 g) obtained in Example 11, phenylboronic acid (0.28 g) and anhydrous tetrahydrofuran (80 ml) were heated under reflux for 30 min, and cooled to room temperature. BSTFA (2.1 ml) was added, stirred for 1h at room temperature, and cooled to -10∼-5°C. Borane-dimethyl sulfide complex (0.8ml, 0.94%) was added dropwise, and upon addition, warmed to 10∼15°C for reacting for 3.5h. The reaction was monitored by HPLC and the conversion rate was 82%. Afterwards, hydrochloric acid (2 N, 4.8 ml) was added dropwise and water (160 ml) was added. The solvent was removed under reduced pressure and then stirred at room temperature for 24 h. The residue was loaded onto a preparative column, and eluted with 22% acetonitrile / water (0.15% acetic acid). Collections rich in products were pooled, diluted twice with water, loaded on a preparative column, and eluted with 90% acetonitrile / water (0.15% acetic acid). Fractions were collected and diluted twice with water, loaded on a preparative column, and eluted with 90% acetonitrile / water (0.15% acetic acid). Fractions were collected and lyophilized to give 1.42 g of sample containing the compound of formula X2b. The content of the compound of formula XI2b was determined as 0.09% by HPLC.

### Example 18 (Preparing the compound of formula III from the compound of formula XI2b)

Under an atmosphere of nitrogen, the compound of formula X2b (1.0 g) obtained in Example 17 was dissolved in methanol (4.2 ml), and cooled to -10 to -5°C. Ethylenediamine (4.2 ml) was added dropwise. Upon addition, the reaction was warmed to room temperature for 48 h, and the reaction conversion rate was determined as 99% by HPLC monitoring. The reaction system was added dropwise into a solution of glacial acetic acid solution (8.3 ml) in water (18.5 ml), then diluted twice with water, loaded onto a preparative column and eluted with 22% acetonitrile / water (0.15% acetic acid). Collections rich in products were pooled, diluted twice with water, loaded on a preparative column, and eluted with 90% acetonitrile / water (0.15% acetic acid). Fractions were collected and concentrated to dryness under reduced pressure. The above concentrate was dissolved in a solution (ethanol / water / acetic acid = 210.0/19.7/1.0, v/v/v, 10 ml), and ethyl acetate (14 ml) was added dropwise at 5 to 20°C for crystallization. White crystalline solids (caspofungin acetate, 0.72 g) were obtained by filtration, the purity of which was determined as 99.76% by HPLC, and the content of the compound of formula IV was 0.06%.

### Comparative Example 3 (Preparation of caspofungin acetate without a compound of formula IV according to the method of CN102070707A)

Caspofungin acetate with less than 0.05% of the compound of formula IV was prepared according to the method disclosed in Examples of CN102070707A, and results showed that about 1 g of caspofungin acetate was prepared, which was purified through preparative HPLC (containing RP C-18 resin filler) with acetic acid and acetonitrile buffer, about 6 L of organic solvent, acetonitrile was used, and the used production equipment and resin filler are very expensive.

However, according to Examples 4, 5 and 6, it was found that about 0.3 L of solvent was used for preparing 1 g of the compound of the formula I in which the content of the impurity, the compound of formula II, was 0.1% or less, therefore, caspofungin acetate can be directly prepared in which the content of the compound of formula IV was less than 0.05%, and it is not necessary to use preparative HPLC for purification.

### Comparative Example 4 (Preparation of caspofungin acetate intermediate without a compound of formula IV according to the method of CN102947327A)

Caspofungin acetate intermediate with low content of serine analog caspofungin intermediate was prepared according to methods disclosed in Examples of CN102947327A. When Caspofungin acetate intermediate with less than 0.1% of serine analog caspofungin intermediate was prepared according to methods described in CN102947327A, the yield was only 20% to 40%, and the value for industrial production was very small.

## Claims

1. A process for preparing a composition comprising a compound of formula I and a compound of formula II, comprising steps of:
(a) dissolving the crude compound of formula I in an aqueous solution of an organic solvent (i);
(b) precipitating solids from the solution by cooling and / or adding an organic solvent (ii) to obtain a suspension containing the compound of formula I;
(C) obtaining composition A containing the compound of formula I and the compound of formula II by centrifugation or filtration;
wherein in step (a), the organic solvent (i) is selected from one or more of methanol, ethanol, n-propanol, isopropanol, isobutanol, n-butanol, and acetone;
in step (b), the organic solvent (ii) is selected from one or more of C₃₋₇ ester, hexane, n-heptane, n-pentane, and dichloromethane;

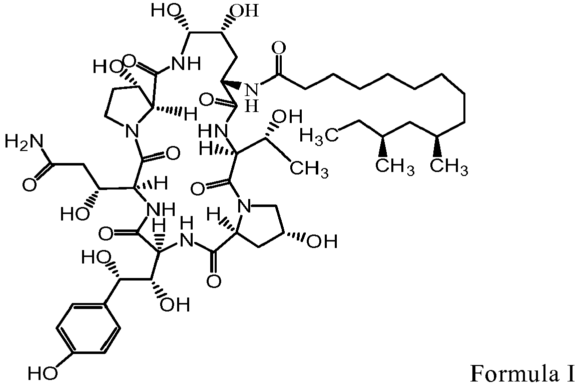

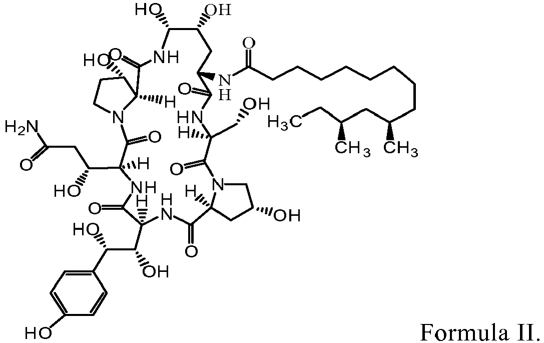

2. The process of claim 1, wherein, in step (a), the volume percentage of water in the aqueous solution of organic solvent (i) is from 8% to 30%; or
in step (b), the organic solvent (ii) is selected from one or more of ethyl acetate, isopropyl acetate, n-hexane; or
steps (a) - (c) may be repeated one or more times.

3. The process of claim 1, wherein, in step (a), the volume percentage of water in the aqueous solution of organic solvent (i) is from 10% to 25%.

4. The process of claim 3, wherein, in step (a), the volume percentage of water in the aqueous solution of organic solvent (i) is from 12% to 22%.

5. The process of claim 4, wherein, in step (a), the volume percentage of water in the aqueous solution of organic solvent (i) is from 16% to 22%.

6. A suspension containing the compound of formula I obtained by the process of any one of claims 1-5, wherein the diameter of the solid particles in the suspension is greater than or equal to 0.1 µm.

7. The suspension of claim 6, wherein the diameter of the solid particles in the suspension is greater than or equal to 0.2 µm.

8. The suspension of claim 7, wherein the diameter of the solid particles in the suspension is greater than or equal to 0.3 µm.

9. The process of claim 1, wherein, after step (c), step (d) is further included:
(d) composition A obtained in step (c) is further dried to obtain a dry composition B.

10. The process of claim 9, wherein the moisture content of composition B is less than or equal to 5%; or
in the dry composition B, the dry content of the compound of formula I is more than 95%; and the dry content of the compound of formula II is from 0.0001% to 2.0%.

11. The process of claim 9, wherein, in the dry composition B, the dry content of the compound of formula I is from 0.0001 % to 0.49 %.

12. The process of claim 11, wherein, in the dry composition B, the dry content of the compound of formula I is from 0.0001% to 0.2%.

13. The process of claim 12, wherein, in the dry composition B, the dry content of the compound of formula I is from 0.0001% to 0.1%.

## Patentansprüche

1. Verfahren zur Herstellung einer Zusammensetzung, umfassend eine Verbindung der Formel I und eine Verbindung der Formel II, wobei das Verfahren folgende Schritte umfasst:
(a) Lösen der Rohverbindung der Formel I in einer wässrigen Lösung eines organischen Lösungsmittels (i);
(b) Ausfällen von Feststoffen aus der Lösung durch Abkühlen und/oder Zusetzen eines organischen Lösungsmittels (ii), um eine die Verbindung der Formel I enthaltende Suspension zu erhalten;
(c) Erhalten von die Verbindung der Formel I und die Verbindung der Formel II enthaltende Zusammensetzung A durch Zentrifugieren oder Filtration;
wobei das organische Lösungsmittel (i) in Schritt (a) aus einem oder mehreren von Methanol, Ethanol, n-Propanol, Isopropanol, Isobutanol, n-Butanol und Aceton ausgewählt ist;
das organische Lösungsmittel (ii) in Schritt (b) aus einem oder mehreren von C₃₋₇-Ester, Hexan, n-Heptan, n-Pentan und Dichlormethan ausgewählt ist;

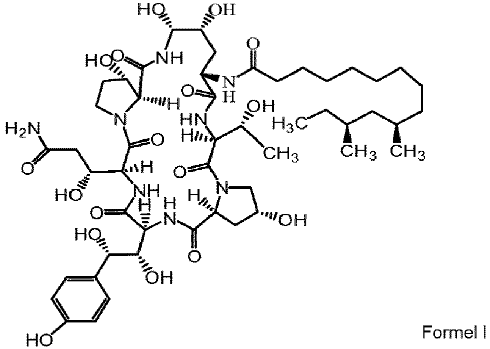

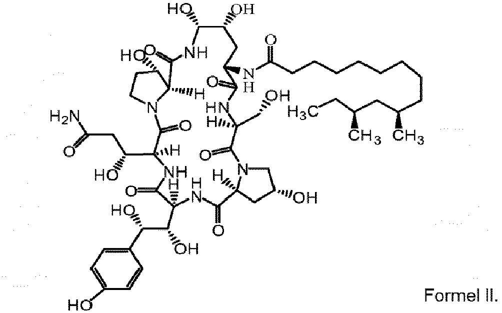

2. Verfahren nach Anspruch 1, wobei der Volumenanteil von Wasser in der wässrigen Lösung von organischem Lösungsmittel (i) in Schritt (a) 8 % bis 30 % beträgt; oder
das organische Lösungsmittel (ii) in Schritt (b) aus einem oder mehreren von Ethylacetat, Isopropylacetat, n-Hexan ausgewählt ist; oder
Schritte (a) - (c) ein oder mehrere Male wiederholt werden können.

3. Verfahren nach Anspruch 1, wobei der Volumenanteil von Wasser in der wässrigen Lösung von organischem Lösungsmittel (i) in Schritt (a) 10 % bis 25 % beträgt.

4. Verfahren nach Anspruch 3, wobei der Volumenanteil von Wasser in der wässrigen Lösung von organischem Lösungsmittel (i) in Schritt (a) 12 % bis 22 % beträgt.

5. Verfahren nach Anspruch 4, wobei der Volumenanteil von Wasser in der wässrigen Lösung von organischem Lösungsmittel (i) in Schritt (a) 16 % bis 22 % beträgt.

6. Suspension, enthaltend die Verbindung der Formel I, erhalten durch ein Verfahren nach einem der Ansprüche 1 bis 5, wobei der Durchmesser der festen Teilchen in der Suspension größer oder gleich 0,1 [µm ist.

7. Suspension nach Anspruch 6, wobei der Durchmesser der festen Teilchen in der Suspension größer oder gleich 0,2 [µm ist.

8. Suspension nach Anspruch 7, wobei der Durchmesser der festen Teilchen in der Suspension größer oder gleich 0,3 [µm ist.

9. Verfahren nach Anspruch 1, wobei nach Schritt (c) weiters Schritt (d) folgt:
(d) Zusammensetzung A, erhalten in Schritt (c), wird weiter getrocknet, um eine trockene Zusammensetzung B zu erhalten.

10. Verfahren nach Anspruch 9, wobei der Feuchtigkeitsgehalt von Zusammensetzung B kleiner oder gleich 5 % ist; oder
der Trockengehalt der Verbindung der Formel I in der trockenen Zusammensetzung B größer als 95 % ist; und der Trockengehalt der Verbindung der Formel II 0,0001 % bis 2,0 % beträgt.

11. Verfahren nach Anspruch 9, wobei der Trockengehalt der Verbindung der Formel I in der trockenen Zusammensetzung B 0,0001 % bis 0,49 % beträgt.

12. Verfahren nach Anspruch 11, wobei der Trockengehalt der Verbindung der Formel I in der trockenen Zusammensetzung B 0,0001 % bis 0,2 % beträgt.

13. Verfahren nach Anspruch 12, wobei der Trockengehalt der Verbindung der Formel I in der trockenen Zusammensetzung B 0,0001 % bis 0,1 % beträgt.

## Revendications

1. Procédé de préparation d'une composition comprenant un composé de formule I et un composé de formule II, comprenant les étapes consistant à :
(a) dissoudre le composé brut de formule I dans une solution aqueuse d'un solvant organique (i) ;
(b) précipiter des solides à partir de la solution en refroidissant et/ou en ajoutant un solvant organique (ii) pour obtenir une suspension contenant le composé de formule I ;
(c) obtenir la composition A contenant le composé de formule I et le composé de formule II par centrifugation ou filtration ;
dans lequel dans l'étape (a), le solvant organique (i) est choisi parmi un ou plusieurs parmi méthanol, éthanol, n-propanol, isopropanol, isobutanol, n-butanol et acétone ;
à l'étape (b), le solvant organique (ii) est choisi parmi un ou plusieurs parmi ester en C₃ à C₇, hexane, n-heptane, n-pentane et dichlorométhane ;

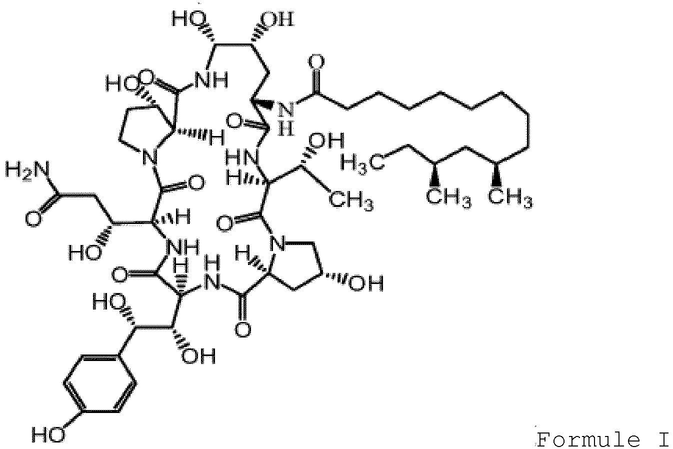

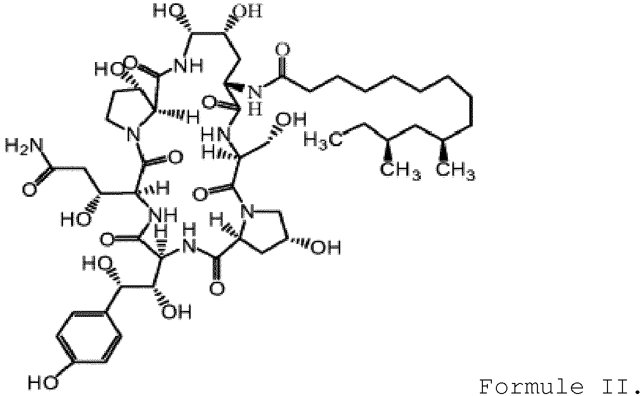

2. Procédé selon la revendication 1, dans lequel, à l'étape (a), le pourcentage en volume d'eau dans la solution aqueuse de solvant organique (i) est compris entre 8 % et 30 % ; ou
à l'étape (b), le solvant organique (ii) est choisi parmi un ou plusieurs parmi acétate d'éthyle, acétate d'isopropyle, n-hexane ; ou
les étapes (a) à (c) peuvent être répétées une ou plusieurs fois.

3. Procédé selon la revendication 1, dans lequel, à l'étape (a), le pourcentage en volume d'eau dans la solution aqueuse de solvant organique (i) est compris entre 10 % et 25 %.

4. Procédé selon la revendication 3, dans lequel, à l'étape (a), le pourcentage en volume d'eau dans la solution aqueuse de solvant organique (i) est compris entre 12 % et 22 %.

5. Procédé selon la revendication 4, dans lequel, à l'étape (a), le pourcentage en volume d'eau dans la solution aqueuse de solvant organique (i) est compris entre 16 % et 22 %.

6. Suspension contenant le composé de formule I obtenu par le procédé selon l'une quelconque des revendications 1 à 5, dans laquelle le diamètre des particules solides dans la suspension est supérieur ou égal à 0,1 µm.

7. Suspension selon la revendication 6, dans laquelle le diamètre des particules solides dans la suspension est supérieur ou égal à 0,2 µm.

8. Suspension selon la revendication 7, dans laquelle le diamètre des particules solides dans la suspension est supérieur ou égal à 0,3 µm.

9. Procédé selon la revendication 1, dans lequel, après l'étape (c), l'étape (d) est en outre incluse :
(d) la composition A obtenue à l'étape (c) est en outre séchée pour obtenir une composition sèche B.

10. Procédé selon la revendication 9, dans lequel la teneur en humidité de la composition B est inférieure ou égale à 5 % ; ou
dans la composition sèche B, la teneur en matière sèche du composé de formule I est supérieure à 95 % ; et la teneur en matière sèche du composé de formule II est comprise entre 0,0001 % et 2,0

11. Procédé selon la revendication 9, dans lequel, dans la composition sèche B, la teneur en matière sèche du composé de formule I est comprise entre 0,0001 % et 0,49

12. Procédé selon la revendication 11, dans lequel, dans la composition sèche B, la teneur en matière sèche du composé de formule I est comprise entre 0,0001 % et 0,2

13. Procédé selon la revendication 12, dans lequel, dans la composition sèche B, la teneur en matière sèche du composé de formule I est comprise entre 0,0001 % et 0,1
